# FASCICULE DE BREVET EUROPEEN

(11) **EP 2 286 273 B1**
(45) Date de publication et mention de la délivrance du brevet: **24.10.2012**
(21) Numéro de dépôt: 09750051.6
(22) Date de dépôt: 07.05.2009
(51) Int. Cl.: G01T 1/02, G01T 1/29

(54) **PROCEDE ET DISPOSITIF DE MESURE EN TEMPS REEL D'UNE DOSE LOCALE LORS DU BOMBARDEMENT D'UNE CIBLE PAR DES HADRONS AU MOYEN DE RAYONS GAMMA PROMPTS**
VERFAHREN UND VORRICHTUNG ZUR ECHTZEITMESSUNG EINER LOKALEN DOSIS NACH DER BOMBARIDERUNG EINES ZIELS MIT HADRONEN MITTELS PROMPTER GAMMASTRAHLUNG
METHOD AND DEVICE FOR REAL-TIME MEASUREMENT OF A LOCAL DOSE UPON BOMBARDMENT OF A TARGET BY HADRONS BY MEANS OF PROMPT GAMMA RAYS

(30) Priorité: 07.05.2008 FR 0853038
(43) Date de publication de la demande: 23.02.2011
(73) Titulaire: Centre National de la Recherche Scientifique - CNRS, 75794 Paris Cedex 16 (FR); Universite Claude Bernard Lyon 1, 69100 Villeurbanne (FR)
(72) Inventeur: TESTA, Etienne, F-69007 Lyon (FR); RAY, Cédric, F-69006 Lyon (FR); FREUD, Nicolas, F-69006 Lyon (FR)
(74) Mandataire: Cabinet Plasseraud
(86) Numéro de dépôt international: PCT/FR2009/050848
(87) Numéro de publication internationale: WO 2009/141570

(56) Documents cités:
- KR-B1- 100 783 506
- MIN CHUL-HEE ET AL: "Prompt gamma measurements for locating the dose falloff region in the proton therapy" APPLIED PHYSICS LETTERS, AIP, AMERICAN INSTITUTE OF PHYSICS, MELVILLE, NY, vol. 89, no. 18, 2 novembre 2006 (2006-11-02), pages 183517-183517, XP012086792 ISSN: 0003-6951 cité dans la demande
- SCROGGS R J ET AL: "A multicrystal gamma-ray spectrometer with time-of-flight rejection of neutron-induced background" INTERNATIONAL SYMPOSIUM ON PLASMA PHENOMENA AND MEASUREMENT OCT. 1963 SAN DIEGO, CA, USA, vol. NS-11, no. 1, janvier 1964 (1964-01), pages 365-373, XP002513651 IEEE Transactions on Nuclear Science USA
- PAULO CRESPO ET AL: "Direct time-of-flight for quantitative, real-time in-beam PET: a concept and feasibility study; Direct time-of-flight for quantitative, real-time in-beam PET" PHYSICS IN MEDICINE AND BIOLOGY, TAYLOR AND FRANCIS LTD. LONDON, GB, vol. 52, no. 23, 7 décembre 2007 (2007-12-07), pages 6795-6811, XP020127262 ISSN: 0031-9155

## Description

La présente invention porte sur un procédé et dispositif de mesure en temps réel d'une dose locale lors du bombardement d'une cible par des hadrons. Elle s'applique notamment aux traitements de patients par des hadrons, dits hadronthérapie. L'hadronthérapie a été proposée par Robert R. Wilson en 1946 et se fonde sur les caractéristiques de dépôt d'énergie des ions chargés dans la matière. Elle vise notamment à améliorer le traitement des cancers grâce à une excellente précision balistique et à une efficacité biologique optimale, proche de 1 dans les tissus sains et de l'ordre de 2 à 3 dans le volume tumoral (l'efficacité biologique d'un rayonnement est définie comme le rapport des doses délivrées avec des rayons X et avec le rayonnement considéré pour obtenir un même effet biologique, typiquement un taux de survie d'une population cellulaire de 10 %).

En effet, contrairement aux rayonnements conventionnels, tels que les photons (X ou gamma) ou les électrons, dont le profil de dose délivrée aux tissus diminue progressivement avec la profondeur traversée, celui des ions permet un dépôt de dose élevé en fin de parcours (dénommé pic de Bragg) alors que la dose déposée en amont (correspondant à la région dite du plateau) est beaucoup plus faible. La position en profondeur du pic de Bragg, qui est contrôlée par l'énergie incidente du faisceau de hadrons chargés, peut être modifiée, permettant ainsi de déposer le maximum de l'énergie au sein d'un volume cible circonscrit, notamment une tumeur, tout en épargnant les tissus sains en amont et en aval.

De manière connue en soi les hadrons ionisés sont accélérés par un cyclotron ou un synchrotron et l'énergie de la particule à la sortie de l'accélérateur détermine la profondeur de pénétration et la position du maximum de l'efficacité biologique de l'irradiation.

Grâce à ces propriétés, alliées à une faible diffusion latérale, la dose déposée dans les tissus par des hadrons chargés peut être confinée avec une précision nettement plus grande qu'en radiothérapie conventionnelle.

Parmi les hadrons, on choisit de préférence des hadrons légers tels que les protons ou les ions carbone. Les ions carbone sont particulièrement avantageux car ils présentent une balistique sensiblement meilleure que celle des protons (moindre dispersion latérale) et une efficacité biologique optimale

L'interaction des hadrons avec les tissus peut, lorsqu'il y a collision inélastique entre les noyaux projectile et cible, créer des phénomènes de fragmentation des hadrons qui produisent notamment des noyaux instables, des rayonnements gamma et des neutrons.

Par facilité de langage, on peut employer le terme « gamma » pour désigner un rayon gamma.

Selon un procédé connu, l'émission de positons (par exemple émis par des noyaux ¹¹C dans le cas d'un faisceau d'ions ¹²C⁶⁺), est utilisée pour mesurer et/ou visualiser la distribution de dose issue de l'interaction entre les hadrons et la cible. On peut notamment utiliser des techniques de Tomographie par Emission de Positons (TEP) à cette fin. Cependant, cette technique présente l'inconvénient d'être une mesure a posteriori qui ne permet pas de suivre l'évolution des doses reçues pendant le traitement.

En outre, il a été noté que la distribution des particules émettrices de positons dans la cible n'est pas toujours corrélable de manière fiable avec la distribution de dose dans la cible.

Afin de pallier ces inconvénients, une technique de mesure de rayons gamma prompts a été proposée par Min et al. pour contrôler le parcours des protons lors d'une irradiation (Chul-Hee Min and Chan Hyeong Kim, Min-Young Youn, Jong-Won Kim "Prompt gamma measurements for locating the dose falloff region in the proton therapy", Applied Physics Letters 89, 183517 (2006)).

Selon cette technique, un scanner à rayon gamma prompts est mis en oeuvre, qui comprend trois couches d'écrans entourant un détecteur pour faire barrière aux neutrons produits par la fragmentation nucléaire. Une première couche de paraffine modère les neutrons de haute énergie, puis une couche de poudre de B₄C permet de capturer une bonne partie des neutrons et le détecteur est finalement disposé au sein d'une couche de plomb qui bloque les rayonnements gamma non désirés. La longueur totale des couches de protection d'un tel scanner atteint environ 70 cm. Il en résulte un appareil encombrant, peu maniable et de faible efficacité.

Le but de la présente invention est de remédier aux inconvénients ci-dessus. Par conséquent l'invention a pour but de proposer un procédé de mesure, notamment pour la hadronthérapie, qui permette une mesure locale en temps réel et assure une bonne maniabilité des moyens mis en oeuvre. Il est en outre susceptible d'être mis en oeuvre pour mesurer la distribution spatiale (suivant 2 ou 3 dimensions) de doses locales reçue par une pluralité de régions d'une cible lors du bombardement de ladite cible par un faisceau de hadrons incidents.

Ce but est atteint par le procédé de mesure en temps réel d'une dose locale reçue par une région d'une cible lors du bombardement de ladite cible par un faisceau de hadrons incidents qui conduit à une fragmentation nucléaire dans ladite cible et génère au moins des rayonnements gamma prompts et des neutrons, où l'on mesure les photons et les particules émises par la cible en collimatant la région de la cible et en disposant un détecteur à une distance L de la région à mesurer de la cible, ledit détecteur étant associé à des moyens de mesure en énergie et en temps de vol des photons et des particules, et où l'on détermine le nombre de rayons gamma prompts reçus par ledit détecteur en sélectionnant les événements enregistrés correspondant à des particules d'énergie E, supérieure ou égale à une énergie seuil Eₛ, puis en sélectionnant parmi ces événements ceux compris dans une plage de temps dt sensiblement centrée sur le temps de parcours de la distance L par les rayons gamma prompts, et où la distance L est choisie pour permettre de discriminer les rayons gamma prompts des neutrons grâce à leur différence de vitesse de propagation et où on met en oeuvre un système de détection bidirectionnel de particules chargées, disposé dans le faisceau de hadrons incidents avant la cible de manière à obtenir la position transverse des hadrons incidents.

On entend par « les événements enregistrés correspondant à des particules d'énergie E » les événements pour lesquels l'énergie déposée dans le détecteur a l'énergie E mentionnée.

On entend par « une information spatiale sur les rayons gamma prompts » une information spatiale sur lieux d'émission desdits rayons gamma prompts.

Le système de détection bidirectionnel de particules chargées, disposé dans le faisceau de hadrons incidents avant la cible de manière à obtenir la position transverse des hadrons incidents permet d'obtenir une information spatiale sur les rayons gamma prompts.

Le détecteur associé à des moyens de mesure en énergie et en temps de vol de particules fait partie d'un système de détection de rayons gamma prompts comprenant également des moyens de traitement du signal reçu par ledit détecteur.

Grâce à l'utilisation de moyens de mesure en énergie et en temps de vol combinés avec le choix de domaine d'énergie et d'une distance entre le détecteur et la région à mesurer de la cible permettant la discrimination par temps de vol des rayons gamma prompts d'avec les neutrons, on peut s'affranchir d'une partie importante des couches de protection nécessaires selon la technique de Min et al. On peut ainsi obtenir un dispositif de mesure en temps réel peu volumineux et très maniable. Les inventeurs ont en effet pu montrer que les événements comptabilisés au-delà d'une énergie seuil Eₛ sont susceptibles d'être discriminés en temps de vol afin d'identifier les événements relatifs à la détection de rayons gamma prompts, permettant ainsi une mesure locale de la dose reçue par une cible. En outre le système de détection bidirectionnel de particules chargées permet de repérer spatialement la particule et de déterminer notamment sa position transverse par rapport au faisceau de hadrons incidents. Il est ainsi possible d'obtenir des informations bi, voire tridimensionnelles relatives aux événements enregistrés. Parmi les systèmes de détection bidirectionnels de particules chargées, on peut choisir un profileur de faisceau.

Les inventeurs ont pu montrer que la position du système de détection bidirectionnel de particules chargées, disposé dans le faisceau de hadrons incidents avant la cible, est particulièrement avantageuse. En effet, la position transverse des ions dans la cible est sensiblement la même que celle des ions dans le faisceau incident. Cette identité de position transverse, à quelques millimètres près, permet de disposer simplement dans le faisceau incident le système de détection bidirectionnel et d'en déduire une information permettant de localiser le point d'émission du rayon gamma prompt dans la cible.

Selon un autre mode de réalisation, on mesure en temps réel une distribution spatiale tridimensionnelle de doses locales reçues par une pluralité de régions d'une cible en déplaçant spatialement la cible ou le détecteur par rapport respectivement au détecteur ou à la cible en mettant en oeuvre le système de détection bidirectionnel de particules chargées, disposé dans le faisceau de hadrons incidents avant la cible de manière à obtenir la position transverse des hadrons incidents.

On obtient ainsi avantageusement une mesure des doses reçues par la cible en différents points de son volume. Une telle mesure permet un contrôle précis d'un traitement par hadronthérapie grâce à la mesure tridimensionnelle de la distribution d'émission des rayons gamma prompts qui est étroitement corrélée à la distribution de dose. Dans ce mode de réalisation, le système de détection des rayons gamma prompts est associé à un détecteur de particules chargées, inséré dans le faisceau, en amont de la cible. On peut ainsi obtenir les trois coordonnées du point d'émission des rayons gamma prompts : les coordonnées en x et y (dans le plan transverse à la direction du faisceau) sont fournies par le détecteur de particules chargées et la coordonnée en z est fournie par le système de détection des rayons gamma prompts, où z est une direction parallèle à celle du faisceau de hadrons incidents.

Selon un mode de réalisation de la présente invention, on mesure en temps réel une distribution longitudinale de doses locales reçues par une pluralité de régions d'une cible en déplaçant longitudinalement la cible ou le détecteur par rapport respectivement au détecteur ou à la cible.

On entend par « longitudinale » une direction parallèle à celle du faisceau de hadrons incidents. On peut ainsi déterminer les doses reçues par la cible en fonction de la profondeur de pénétration dans la cible.

Selon un mode de réalisation de la présente invention, on dispose dans le faisceau de hadrons incidents un système de détection de particules chargées susceptible de fournir un étiquetage en temps des hadrons incidents et on introduit les paramètres correspondants dans les moyens de mesure en temps de vol de particules. On établit ainsi une corrélation temporelle entre le système de détection de rayons gamma prompts et le système de détection de particules chargées pour l'étiquetage de manière à donner aux moyens de mesure en temps de vol une valeur de temps initial à partir duquel s'effectue le comptage. Cette configuration est particulièrement bien adaptée au cas où les hadrons sont émis sous la forme d'un faisceau temporellement sensiblement continu, comme par exemple fourni par un synchrotron. Le système de détection de particules chargées pour l'étiquetage peut être par exemple un hodoscope de fibres scintillantes ou un détecteur diamant polycristallin.

Le système de détection bidirectionnel de particules chargées, par exemple un hodoscope, peut également servir de système d'étiquetage.

On note que d'autres moyens sont susceptibles de permettre l'initialisation en temps, comme par exemple l'exploitation des variations de flux d'un faisceau de hadrons incidents notamment quand ce faisceau est pulsé.

A titre d'exemple, on peut citer des structures temporelles de paquets d'ions bien adaptées à de telles mesures, où la durée d'émission du paquet d'ions est de l'ordre de 1 ns à 5 ns toutes les dizaines à quelques dizaines de ns.

La cible pourrait être un patient, et la région mesurée de ladite cible pourrait être une zone de tumeur cancéreuse (ou les tissus sains situés en amont et en aval).

Selon un mode de réalisation de la présente invention, la distance L est supérieure ou égale à 40 cm, par exemple supérieure ou égale à 60 cm, et par exemple inférieure ou égale à 100 cm. Une distance supérieure ou égale à 40 cm est en effet avantageuse car elle permet de séparer temporellement les rayons gamma prompts des neutrons. En effet, la vitesse de propagation des rayons gamma prompts est de 30 cm/ns (nanoseconde) alors que celle des neutrons les plus rapides n'excède généralement pas celle des hadrons incidents (au maximum 2/3 de la vitesse de la lumière, soit 20 cm/ns). Il en résulte qu'un neutron mettra au minimum 50% de temps de plus que le rayon gamma prompt à parcourir la distance L. Les moyens de mesure de temps de vol permettent ainsi de séparer les particules (gamma prompts) à comptabiliser pour mesurer une dose locale d'avec les particules (neutrons et rayons gamma diffusés par les matériaux environnants) qui ne peuvent être précisément corrélées avec la mesure d'une dose locale. Une telle différence temporelle permet une sélection satisfaisante des événements à considérer. La distance L peut être augmentée, permettant d'accroître le décalage temporel entre un événement lié à un rayon gamma prompt d'avec un événement lié à un neutron. Cependant la surface de détecteurs nécessaire pour maintenir constant le nombre de photons détectés pour un moyen de collimation donné est proportionnelle à la distance L. De préférence on utilisera une distance L inférieure ou égale à 1 m.

Selon d'autres modes de réalisation de l'invention qui peuvent être combinés entre eux :
- l'énergie seuil Eₛ est supérieure ou égale à 500 keV, de préférence inférieure ou égale à 5 MeV, voire à 2 MeV, par exemple de l'ordre de 1 MeV ;
- la plage de temps dt est comprise entre 1 et 15 ns (nano seconde), par exemple de l'ordre de 7 ns ;
- on déplace le détecteur longitudinalement et/ou angulairement par rapport à la cible.

Selon un mode de réalisation de la présente invention on dispose un collimateur physique comprenant une fente entre la cible et le détecteur associé aux moyens de mesure en énergie et en temps de vol où l'axe de la fente est perpendiculaire au faisceau de hadrons incidents. Un collimateur physique peut par exemple être constitué de blocs de plomb. Le collimateur est disposé par exemple à une distance D de la cible et présente une fente de largeur d. Ces paramètres D et d peuvent être ajustés de façon à couvrir une section de l'épaisseur de la cible qui définit la résolution spatiale de la région à mesurer de la cible. De préférence cette résolution spatiale est inférieure ou égale à 1 cm.

Le détecteur pourrait être associé à des moyens de collimation électroniques. Un tel détecteur associé à des moyens de collimation électronique peut par exemple faire partie d'une caméra Compton.

L'invention vise également un procédé de mesure en temps réel d'une pluralité de doses locales reçues dans une pluralité de régions d'une cible en mettant en oeuvre un ou plusieurs des modes de réalisation du procédé ci-dessus, grâce à une pluralité de détecteurs associés à des moyens de mesure en énergie et en temps de vol répartis spatialement le long et/ou autour de la cible.

L'invention porte également sur un dispositif de mesure en temps réel d'une dose locale reçue par une région d'une cible lors du bombardement de ladite cible par un faisceau de hadrons incidents comprenant :
- un détecteur associé à des moyens de mesure en énergie et en temps de vol de particules ;
- des moyens de collimation de la région de la cible ;
- des moyens permettant de sélectionner les événements correspondant à des particules d'énergie E, supérieure à une valeur seuil Eₛ enregistrée grâce au détecteur ;
- des moyens permettant de sélectionner parmi ces derniers événements des événements compris dans une plage de temps dt donnée et où le détecteur est situé à une distance L de la région à mesurer de la cible choisie de manière à permettre de discriminer des rayons gamma prompts et des neutrons grâce à leur différence de vitesse de propagation ;
- un système de détection bidirectionnel de particules chargées, disposé dans le faisceau de hadrons incidents avant la cible de manière à obtenir la position transverse des hadrons incidents.

Selon différents modes de réalisation dudit dispositif qui peuvent être combinés entre eux :
- la distance L est supérieure ou égale à 40 cm, par exemple supérieure ou égale à 60 cm, et par exemple inférieure ou égale à 100 cm ;
- les moyens de collimation comprennent un collimateur physique comprenant une fente, disposé entre la cible et le détecteur associé aux moyens de mesure en énergie et en temps de vol;
- les moyens de collimation comprennent des moyens de collimation électroniques ;
- le détecteur et les moyens de collimation et/ou la cible sont disposés sur des moyens de déplacement permettant un déplacement longitudinal et/ou angulaire.

L'invention vise également un dispositif de mesure en temps réel d'une pluralité de doses locales reçues par une pluralité de régions d'une cible lors du bombardement de ladite cible par un faisceau de hadrons incidents comprenant une pluralité de dispositifs selon un ou plusieurs des modes de réalisation ci-dessus, répartis spatialement le long et/ou autour de la cible.

L'invention sera mieux comprise à la lecture de la description qui va suivre, donnée uniquement à titre d'exemple et faite en se référant aux dessins annexés dans lesquels :
- la figure 1 est une vue schématique de dessus d'un dispositif selon l'invention ;
- la figure 2 représente schématiquement un spectre en énergie déposée dans le détecteur ;
- la figure 3 représente la variation du nombre d'événements détectés par ion carbone incident de 73 MeV/nucléon dans une cible de PMMA et par intervalle de 1 ns en fonction du temps pour deux domaines d'énergie des rayonnements détectés, tel que mesuré selon l'invention ;
- la figure 4 représente la variation du nombre de particules mesurées en fonction de la position de la cible, selon l'invention ;
- les figures 5 et 6 représentent la variation du taux de rayons gamma prompts au travers de différentes cibles ;
- la figure 7 représente la variation du taux de rayons gamma prompts pour des ions incidents de 292 MeV/nucléon.

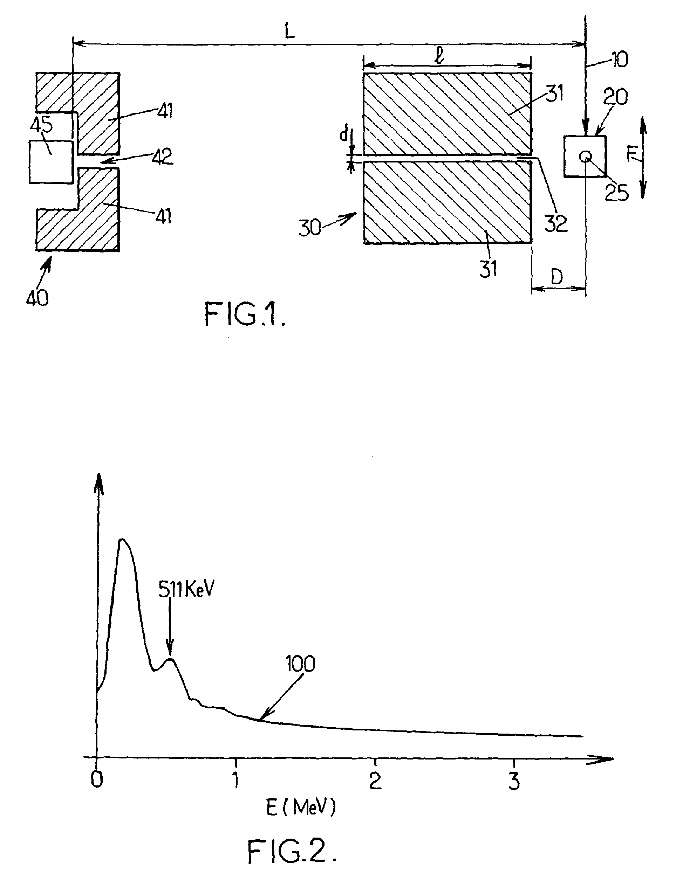

Pour des raisons de clarté, les différents éléments représentés sur la figure 1 ne sont pas nécessairement à l'échelle.

La figure 1 illustre une vue de dessus schématique d'un dispositif selon l'invention.

Un faisceau de hadrons incidents 10 bombarde une cible 20.

On mesure grâce au dispositif selon l'invention la dose locale reçue par la région 25 de la cible 20. La cible 20 peut être déplacée selon la flèche F de manière à mesurer d'autres zones de la cible.

Un collimateur 30 est disposé devant la cible 20 à la distance D de celle-ci. Dans l'exemple représenté, le collimateur est constitué de deux blocs de plomb 31 de longueur *l* espacés de manière à former une fente 32 de largeur d.

Un système de détection 40 est placé derrière le collimateur 30 et comprend un détecteur 45 placé dans l'alignement de la fente 32 du collimateur. Le détecteur est entouré partiellement par des blocs de plomb 41 espacés de manière à laisser une ouverture 42 face au détecteur 45.

Selon un exemple de réalisation de l'invention :
- le détecteur 45 est un détecteur cylindrique de NaI(T1) de 5 cm de hauteur et de 5 cm de diamètre placé à 90° du faisceau de hadrons incidents 10 ;
- le collimateur 30 est constitué de deux blocs de plomb 31 de longueur 1 = 20 cm et disposés de manière à former une fente de largeur d = 2 mm, orthogonale à la direction du faisceau de hadrons, et situé à la distance D = 10 cm de la cible ;
- la distance entre le détecteur 45 et la région 25 à mesurer de la cible 20 est L = 60 cm ;
- la cible est un bloc en PMMA (C₅H₈O₂) de forme cubique, où un côté mesure 10 cm.

Des mesures ont été effectuées avec le dispositif ci-dessus dans les conditions suivantes :
- un faisceau de 73 MeV/u d'ions de ¹³C⁶⁺ est utilisé. Le faisceau est pulsé (environ 1 ns toutes les 80 ns) ;
- l'intensité sur la cible a été fixée à environ 1 nA ;
- un second détecteur de NaI(T1) est utilisé pour mesurer la dose totale et est dépourvu de moyens de collimation et placé à une distance éloignée de la cible pour obtenir un taux de comptage proportionnel à l'intensité du faisceau sans être dépendant de la position de la cible. Ce détecteur a été étalonné avec une cage de Faraday ;
- la mesure de temps est réalisée à l'aide d'un convertisseur temps-numérique qui mesure la différence de temps entre le signal logique délivré par un discriminateur à seuil synchronisé avec la pulsation haute fréquence de l'accélérateur, et le signal logique délivré par un discriminateur à fraction constante déclenché par le signal analogique du détecteur. Le signal analogique de ce détecteur est par ailleurs amplifié à l'aide d'un amplificateur de spectroscopie, et converti par un convertisseur analogique - numérique. L'information relative au temps initial pour le comptage en temps de vol est décelée par le détecteur de rayons gamma prompts 45 et l'information relative au temps final est fournie par le signal haute fréquence de l'accélérateur dans le cas présent de l'utilisation d'un cyclotron.

L'énergie E et le temps t associés à chaque événement sont mesurés en même temps, ainsi que le nombre n d'événements détectés par le second détecteur NaI(T1) durant un essai, qui est proportionnel au nombre d'ions incidents.

La figure 2 représente un spectre d'énergie 100 typique obtenu durant un tel essai. On note que la forme du spectre est sensiblement indépendante des paramètres de configuration. Un seul pic est visible à 511 keV qui correspond principalement à la création de paires par des photons de haute énergie suivie par une annihilation de positon.

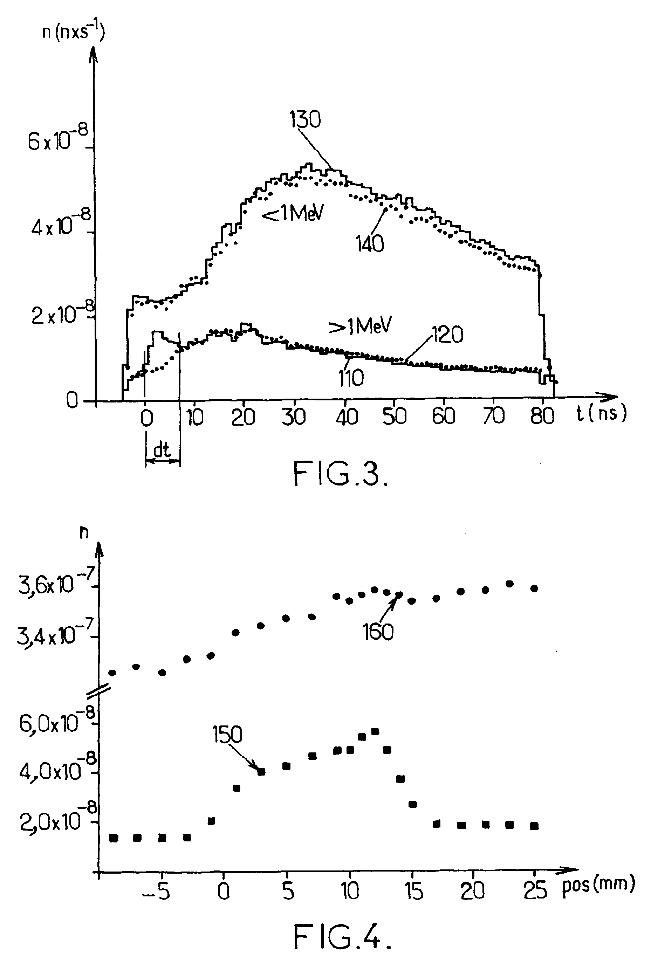

La figure 3 représente des spectres en temps de vol, où l'on porte le nombre d'événements par ion incident et par intervalle de 1 ns (exprimé en ns⁻¹), en fonction du temps t en nanoseconde (ns). L'origine des temps correspond à l'instant de l'impact d'un ion sur la cible.

Les deux spectres 110, 130, en traits continus, représentent des spectres en temps de vol pour une mesure où le collimateur vise une position dans la cible située à 8 mm à l'arrière de la face qui reçoit le faisceau de hadrons incidents. Cette position correspond sensiblement au milieu du parcours moyen d'un ion.

Les deux spectres 120, 140, marqués par des ronds, représentent des spectres en temps de vol pour une mesure où le collimateur vise une position dans la cible située à 26 mm à l'arrière de la face qui reçoit le faisceau de hadrons incidents. Cette position correspond à une distance située à sensiblement 12 mm derrière le pic de Bragg.

Pour chaque position, on comptabilise les événements dont l'énergie détectée est inférieure à 1 MeV (spectres 130 et 140) et ceux dont l'énergie est supérieure à 1 MeV (spectres 110 et 120).

On constate que les spectres 130, 140 sont sensiblement confondus et leur comparaison ne permet pas d'identifier d'événements particuliers. On peut penser que, dans les présentes conditions expérimentales, quand l'énergie est inférieure à 1 MeV, la distribution des événements est totalement dominée par les neutrons. A la différence des spectres précédents, les spectres 110 et 120 diffèrent dans une zone de temps de vol notée dt. On constate en effet que le nombre d'événements comptabilisés est très supérieur pour le spectre 110 comparé au spectre 120. Ces événements peuvent sans ambiguïté être attribués au comptage du rayonnement gamma prompt dans la cible. En effet, la plage dt de temps correspond à une plage de temps s'étendant autour du temps nécessaire à un rayon gamma prompt pour parcourir la distance L = 60 cm, c'est-à-dire de l'ordre de 2 ns. On note que la mesure permet de comptabiliser le nombre de rayons gamma prompts avec une excellente précision en considérant l'ensemble des événements sur la plage de temps dt et que le rapport signal sur bruit est de l'ordre de 1. Il en résulte qu'il est ainsi possible de déterminer une dose locale dans une cible en utilisant à la fois une sélection en énergie et en temps de vol.

La figure 4 représente la variation du nombre d'événements détectés en utilisant les conditions expérimentales ci-dessus en fonction de la distance à la face de la cible qui reçoit le faisceau de hadrons. Les mesures sont obtenues en déplaçant la cible selon la flèche F et en laissant l'ensemble collimateur, dispositif de détection en position constante. La position « pos = O » correspond à une position du collimateur orthogonale au faisceau de hadron et en regard de la face avant de la cible, les positions « pos » négatives correspondent à des mesures du bruit de fond environnant, les positions « pos » positives correspondent à des mesures dans la cible à une distance « pos » de la face avant, où le collimateur demeure en position orthogonale au faisceau de hadron.

Les deux courbes 150 et 160 ont été obtenues après sélection des événements dont l'énergie est supérieure à 1 MeV, dans des conditions similaires d'acquisition.

La courbe 150 reporte les événements détectés pour un comptage en temps de vol entre 2 et 10 ns. La courbe 160 reporte les événements détectés pour un comptage en temps de vol à des temps supérieurs à 10 ns.

On note que la courbe 160 est de variation sensiblement continue et on attribue les événements à la présence de neutrons dont le temps de vol est d'au moins environ 6 ns dans les conditions expérimentales mentionnées.

On note que la courbe 150 présente un pic dans une zone de position sensiblement comprise entre 0 et 15 mm. Ce pic est attribué au comptage des rayons gamma prompts en fonction de l'épaisseur traversée dans la cible. Ces résultats sont tout à fait cohérents avec l'observation de la cible en PMMA qui est jaunie sur une profondeur de 14 mm précisément. On note que le pic de la courbe 150 peut se décomposer en une zone d'événements en nombre croissant modérément, située entre 2 et 10 mm de la face avant de la cible, suivie d'un pic centré autour de 12 à 13 mm de la face avant de la cible. Ce pic est corrélable avec le pic de Bragg du hadron incident qui est à 14 mm.

On démontre donc que dans les conditions de la présente invention, il est possible d'identifier les effets du pic de Bragg dans une cible et de mesurer la quantité de rayons gamma prompts associés, permettant ainsi d'obtenir une mesure corrélable avec la quantité de hadrons reçue en une zone donnée d'une cible. La relation entre parcours et énergie déposée est très bien connue dans la littérature. On peut ainsi obtenir la relation entre le nombre de rayons gamma prompts et la dose locale.

Les résultats rapportés ici et obtenus avec des ions incidents à 73 MeV/nucléon permettent de démontrer que les taux de comptage sont compatibles avec un système de monitorage en temps réel. De plus, grâce à l'exploitation des résultats mesurés pour une gamme d'énergie sélectionnée et pour une fenêtre de temps de vol déterminée, il est possible de s'affranchir d'une partie des dispositifs de protection encombrants mis en oeuvre dans la méthode décrite par Min et al (notamment le blindage contre les neutrons). On peut ainsi proposer des détecteurs légers et peu encombrants, susceptibles d'être associés pour constituer un ensemble de détecteurs et mesurer spatialement avec une grande précision la quantité de rayons gamma prompts émise par une zone d'une cible et déterminer le flux de hadrons reçu localement.

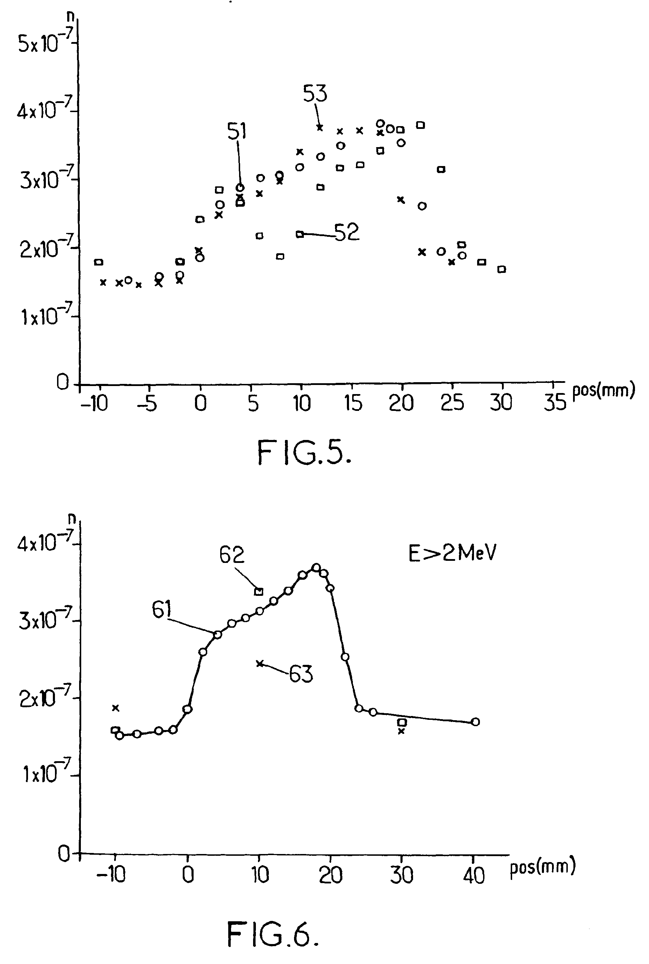

Les figures 5 et 6 reportent la variation du nombre d'événements détectés avec le dispositif précédent dans des conditions expérimentales différentes :
Les ions incidents sont des ions de ¹²C de 95 MeV/nucléon et les événements sélectionnés par temps de vol sont ceux dont l'énergie est supérieure à 2 MeV, obtenus à 90° et avec une largeur de fente de collimation de 2 mm.

Les résultats présentés en figure 5 ont été obtenus avec des cibles de différents matériaux. Les données représentées par des cercles, référencés 51, ont été obtenues avec une cible en PMMA ; celles représentées par des carrés, référencés 52, avec une cible en téflon (masse volumique de 1,9 g/cm³, proche de celle d'un os) ; celles représentées par des croix, référencées 53, avec un matériau équivalent au poumon (masse volumique de 0, 3 g/cm³).

La face avant de la cible de PMMA est située en position pos = 0 et s'étend jusqu'à pos = 33 mm. La face avant de la cible en téflon est située en position pos = 10 mm et s'étend jusqu'à pos = 12,5 mm. La face avant de la cible équivalente au poumon est située en position pos = 6 mm et s'étend jusqu'à pos = 12,5 mm.

On démontre ainsi que le taux de rayons gamma prompts dépend de la densité de matériau observé et qu'il est possible de localiser des inhomogénéités dans la cible traversée grâce à la méthode selon l'invention.

Les résultats présentés en figure 6 ont été obtenus avec des cibles de PMMA de différentes tailles et formes. Les données représentées par des cercles, référencés 61, ont été obtenues avec un cube de 5 cm de côté ; celles représentées par des carrés, référencés 62, avec un cylindre de 2 cm de diamètre ; celles représentées par des croix, référencées 63, avec un cube de 30 cm de côté.

On note que l'atténuation des photons dans une cible volumineuse ne fait pas disparaître le signal. En outre quand la cible est petite, le rapport signal sur bruit diminue, mais reste suffisamment élevé pour fournir en temps réel une information sur le parcours des ions.

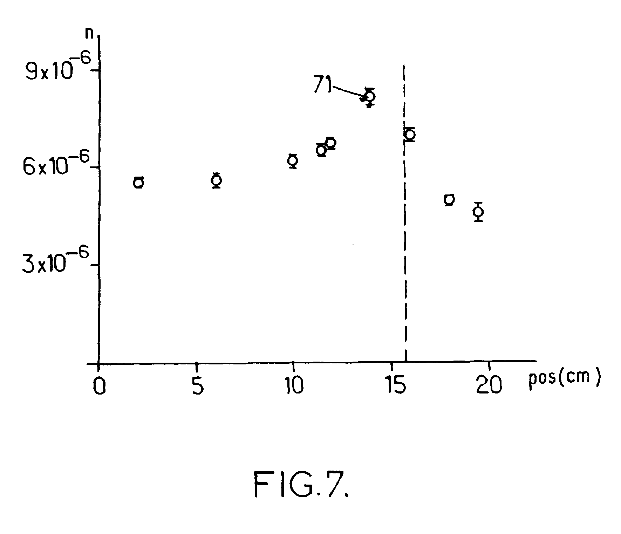

La figure 7 reporte des données expérimentales obtenues dans des conditions où les ions incidents sont des ions ¹²C de 292 MeV/nucléon. Les parcours ainsi obtenus sont similaires à ceux utilisés en hadronthérapie pour atteindre des tumeurs profondes. Les événements sélectionnés par temps de vol sont ceux dont l'énergie est supérieure à 2,5 MeV, la largeur de la fente de collimation est de 1 cm et la cible est de l'eau.

La position calculée du pic de Bragg est représentée en trait pointillé et les taux de comptage des rayons gamma prompts sont représentés par des cercles, référencés 71. Le profil ainsi observé confirme que les rendements mesurés sont compatibles avec le contrôle en ligne et en temps réel d'un traitement par hadronthérapie.

Grâce au procédé de mesure et/ou au dispositif selon l'invention, il est donc possible d'obtenir des moyens de contrôle de hadronthérapie en temps réel, en obtenant une distribution bidirectionnelle, ou de préférence tridimensionnelle de la dose déposée dans le patient, avec une résolution de l'ordre du mm.

On peut estimer que le contrôle temporel peut être effectué à l'échelle de la seconde.

Il est ainsi par exemple possible d'interrompre un traitement en cas de mauvaise distribution de la dose par rapport au plan de traitement.

Il est à noter que divers détecteurs peuvent être utilisés pour mettre en oeuvre la présente invention et que des variantes peuvent être apportées à la structure du dispositif précédemment décrit et à son procédé d'utilisation.

## Revendications

1. Procédé de mesure en temps réel d'une dose locale reçue par une région (25) d'une cible (20) lors du bombardement de ladite cible (20) par un faisceau de hadrons incident (10) qui conduit à une fragmentation nucléaire dans la dite cible (20) et génère au moins des rayonnements gamma prompts et des neutrons, où l'on mesure les particules émises par la cible (20) en collimatant la région (25) de la cible (20) et en disposant un détecteur (45) à une distance L de la région (25) à mesurer de la cible (20), ledit détecteur (45) étant associé à des moyens de mesure en énergie et en temps de vol de particules, et où l'on détermine le nombre de rayons gamma prompts reçus par ledit détecteur (45) en sélectionnant les événements enregistrés correspondant à des particules d'énergie E, supérieure ou égale à une énergie seuil Eₛ, puis en sélectionnant parmi ces événements ceux compris dans une plage de temps dt sensiblement centrée sur le temps de parcours de la distance L par les rayons gamma prompts, et où la distance L est choisie pour permettre de discriminer les rayons gamma prompts des neutrons grâce à leur différence de vitesse de propagation et en ce qu'on met en oeuvre un système de détection bidirectionnel de particules chargées, disposé dans le faisceau de hadrons incidents (10) avant la cible (20) de manière à obtenir la position transverse des hadrons incidents (10).

2. Procédé selon la revendication précédente **caractérisé en ce que** l'on mesure en temps réel une distribution spatiale tridimensionnelle de doses locales reçues par une pluralité de régions (25) d'une cible (20) en déplaçant spatialement la cible (20) ou le détecteur (45) par rapport respectivement au détecteur (45) ou à la cible (20) en mettant en oeuvre ledit système de détection bidirectionnel de particules chargées.

3. Procédé selon l'une quelconque des revendications précédentes **caractérisé en ce que** l'on mesure en temps réel une distribution longitudinale de doses locales reçues par une pluralité de régions (25) d'une cible (20) en déplaçant longitudinalement la cible (20) ou le détecteur (45) par rapport respectivement au détecteur (45) ou à la cible.

4. Procédé selon l'une quelconque des revendications précédentes **caractérisé en ce qu'**on dispose dans le faisceau de hadrons incidents (10) un système de détection de particules chargées susceptible de fournir un étiquetage en temps des hadrons incidents et on introduit les paramètres correspondants dans les moyens de mesure en temps de vol de particules.

5. Procédé selon l'une quelconque des revendications précédentes **caractérisé en ce que** la distance L est supérieure ou égale à 40 cm, par exemple supérieure ou égale à 60 cm, et par exemple inférieure ou égale à 100 cm.

6. Procédé selon l'une quelconque des revendications précédentes **caractérisé en ce que** l'énergie seuil Eₛ est supérieure ou égale à 500 keV, de préférence inférieure ou égale à 5 MeV, voire à 2 MeV, par exemple de l'ordre de 1 MeV.

7. Procédé selon l'une quelconque des revendications précédentes **caractérisé en ce que** la plage de temps dt est comprise entre 1 et 15 ns (nano seconde), par exemple de l'ordre de 7 ns.

8. Procédé selon l'une quelconque des revendications précédentes, **caractérisé en ce que** l'on dispose un collimateur physique (30) comprenant une fente (32) entre la cible (20) et le détecteur associé aux moyens de mesure en énergie et en temps de vol (45) et/ou **en ce que** le détecteur associé aux moyens de mesure en énergie et en temps de vol (45) est associé à des moyens de collimation électronique.

9. Procédé de mesure en temps réel d'une pluralité de doses locales reçues dans une pluralité de régions (25) d'une cible (20) en mettant en oeuvre le procédé selon l'une quelconque des revendications 1 à 8 grâce à une pluralité de détecteurs associés à des moyens de mesure en énergie et en temps de vol (45) répartis spatialement le long et/ou autour de la cible (20).

10. Dispositif de mesure en temps réel d'une dose locale reçue par une région (25) d'une cible (20) lors du bombardement de ladite cible (20) par un faisceau de hadrons incidents (10) comprenant :
- un détecteur (45) associé à des moyens de mesure en énergie et en temps de vol de particules,
- des moyens de collimation (30) de la région (25) de la cible (20),
- des moyens permettant de sélectionner les événements correspondant à des particules d'énergie E, supérieure à une valeur seuil Eₛ enregistrée grâce audit détecteur (45),
- des moyens permettant de sélectionner parmi ces derniers événements des événements compris dans une plage de temps dt donnée et où ledit détecteur (45) est situé à une distance L de la région (25) à mesurer de la cible (20) choisie de manière à permettre de discriminer des rayons gamma prompts et des neutrons grâce à leur différence de vitesse de propagation,
- un système de détection bidirectionnel de particules chargées, disposé dans le faisceau de hadrons incidents (10) avant la cible (20) de manière à obtenir la position transverse des hadrons incidents (10).

11. Dispositif selon la revendication précédente **caractérisé en ce que** la distance L est supérieure ou égale à 40 cm, par exemple supérieure ou égale à 60 cm, et par exemple inférieure ou égale à 100 cm.

12. Dispositif selon l'une quelconque des revendications 10 ou 11 **caractérisé en ce que** les moyens de collimation comprennent un collimateur physique (30) comprenant une fente (32) disposé entre la cible (20) et le détecteur associé aux moyens de mesure en énergie et en temps de vol (45), où l'axe de la fente (32) est perpendiculaire au faisceau de hadrons incidents (10) et/ou en ce que les moyens de collimation comprennent des moyens de collimation électroniques.

13. Dispositif selon l'une quelconque des revendications 10 à 12 **caractérisé en ce que** le détecteur associé aux moyens de mesure en énergie et en temps de vol (45) et les moyens de collimation et/ou la cible (20), sont disposés sur des moyens de déplacement permettant un déplacement longitudinal et/ou angulaire.

14. Dispositif de mesure en temps réel d'une pluralité de doses locales reçues par une pluralité de régions (25) d'une cible (20) lors du bombardement de ladite cible (20) par un faisceau de hadrons incident (10) comprenant une pluralité de dispositifs selon l'une quelconque des revendications 10 à 13, répartis spatialement le long et/ou autour de la cible (20).

## Claims

1. A method for real-time measurement of a local dose received by a region (25) of a target (20) during the bombardment of said target (20) by an incident beam of hadrons (10) which leads to a nuclear fragmentation in said target (20) and generates at least prompt gamma radiations and neutrons, where the particles emitted by the target (20) are measured by collimating the region (25) of the target (20) and by stationing a detector (45) at a distance L from the region (25) to be measured of the target (20), said detector (45) being associated with particle energy and time-of-flight measurement means, and where the number of prompt gamma rays received by said detector (45) is determined by selecting the recorded events corresponding to particles of energy E, greater than or equal to a threshold energy Eₛ, and then by selecting from among these events those included in a time span dt centered substantially on the travel time taken by the prompt gamma rays to traverse the distance L, and where the distance L is chosen so as to make it possible to discriminate the prompt gamma rays from the neutrons by virtue of their difference in propagation speed and in that a bidirectional system for detecting charged particles, stationed in the beam of incident hadrons (10) before the target (20), is implemented in such a way as to obtain the transverse position of the incident hadrons (10).

2. The method as claimed in the preceding claim **characterized in that** a three-dimensional spatial distribution of local doses received by a plurality of regions (25) of a target (20) is measured in real time by spatially displacing the target (20) or the detector (45) in relation respectively to the detector (45) or to the target (20) by implementing said bidirectional system for detecting charged particles.

3. The method as claimed in any one of the preceding claims **characterized in that** a longitudinal distribution of local doses received by a plurality of regions (25) of a target (20) is measured in real time by longitudinally displacing the target (20) or the detector (45) in relation respectively to the detector (45) or to the target.

4. The method as claimed in any one of the preceding claims **characterized in that** a system for detecting charged particles is stationed in the beam of incident hadrons (10), said system being able to provide a time-labeling of the incident hadrons, and the corresponding parameters are introduced into the particle time-of-flight measurement means.

5. The method as claimed in any one of the preceding claims **characterized in that** the distance L is greater than or equal to 40 cm, for example greater than or equal to 60 cm, and for example less than or equal to 100 cm.

6. The method as claimed in any one of the preceding claims **characterized in that** the threshold energy Eₛ is greater than or equal to 500 keV, preferably less than or equal to 5 MeV, or indeed than 2 MeV, for example of the order of 1 MeV.

7. The method as claimed in any one of the preceding claims, **characterized in that** the time span dt lies between 1 and 15 ns (nanoseconds), for example of the order of 7 ns.

8. The method as claimed in any one of the preceding claims, **characterized in that** a physical collimator (30) comprising a slit (32) is stationed between the target (20) and the detector associated with the energy and time-of-flight measurement means (45) and/or **in that** the detector associated with the energy and time-of-flight measurement means (45) is associated with means of electronic collimation.

9. A method for real-time measurement of a plurality of local doses received in a plurality of regions (25) of a target (20) by implementing the method as claimed in any one of claims 1 to 8 by virtue of a plurality of detectors associated with energy and time-of-flight measurement means (45) distributed spatially along and/or around the target (20).

10. A device for real-time measurement of a local dose received by a region (25) of a target (20) during the bombardment of said target (20) by a beam of incident hadrons (10) comprising:
- a detector (45) associated with particle energy and time-of-flight measurement means,
- means of collimating (30) the region (25) of the target (20),
- means making it possible to select the events corresponding to particles of energy E, greater than a threshold value Eₛ recorded by virtue of said detector (45),
- means making it possible to select from among the latter events those events included in a given time span dt and where said detector (45) is situated at a distance L from the region (25) to be measured of the target (20) chosen in such a way as to make it possible to discriminate prompt gamma rays and neutrons by virtue of their difference in propagation speed,
- a bidirectional system for detecting charged particles, stationed in the beam of incident hadrons (10) before the target (20) in such a way as to obtain the transverse position of the incident hadrons (10).

11. The device as claimed in the preceding claim, **characterized in that** the distance L is greater than or equal to 40 cm, for example greater than or equal to 60 cm, and for example less than or equal to 100 cm.

12. The device as claimed in any one of claims 10 or 11, **characterized in that** the collimation means comprise a physical collimator (30) comprising a slit (32), said collimator being stationed between the target (20) and the detector associated with the energy and time-of-flight measurement means (45), where the axis of the slit (32) is perpendicular to the beam of incident hadrons (10) and/or in that the collimation means comprise electronic collimation means.

13. The device as claimed in any one of claims 10 to 12, **characterized in that** the detector associated with the energy and time-of-flight measurement means (45) and the collimation means and/or the target (20), are stationed on displacement means allowing a longitudinal and/or angular displacement.

14. The device for real-time measurement of a plurality of local doses received by a plurality of regions (25) of a target (20) during the bombardment of said target (20) by an incident beam of hadrons (10) comprising a plurality of devices as claimed in any one of claims 10 to 13, distributed spatially along and/or around the target (20).

## Patentansprüche

1. Verfahren zur Echtzeit-Messung von einer lokalen Dosis, die von einem Bereich (25) einer Zielscheibe (20) bei der Bombardierung der Zielscheibe (20) mit einem einfallenden Hadronen-Strahl (10) empfangen wird, was zu einem nuklearen Zerfall in der Zielscheibe (20) führt, und wenigstens schnelle Gammastrahlen und Neutronen erzeugt, wobei die von der Zielscheibe (20) emittierten Teilchen gemessen werden, indem der Bereich (25) der Zielscheibe (20) anvisiert/kollimiert wird, und indem ein Detektor (45) in einem Abstand L von dem zu messenden Bereich (25) der Zielscheibe (20) angeordnet wird, wobei der Detektor (45) Messmitteln für Energie und Flugzeit von Teilchen zugeordnet ist, und wobei die Anzahl von durch den Detektor (45) empfangenen schnellen Gammastrahlen bestimmt wird, indem die aufgezeichneten Ereignisse ausgewählt werden, welche Teilchen mit einer Energie E entsprechen, die größer oder gleich einer Schwellen-Energie Eₛ ist, dann unter diesen Ereignissen diejenigen ausgewählt werden, die in einem Zeit-Bereich dt liegen, der im Wesentlichen auf die Laufzeit der schnellen Gammastrahlen für den Abstand L zentriert ist, und wobei der Abstand L ausgewählt ist, um zu ermöglichen, die schnellen Gammastrahlen von Neutronen zu unterscheiden, dank deren Unterschied in der Ausbreitungsgeschwindigkeit, und indem ein bidirektionales System zur Detektion von geladenen Teilchen eingesetzt wird, welches in dem Strahl von einfallenden Hadronen (10) vor der Zielscheibe (20) angeordnet wird, um die transversale Position der einfallenden Hadronen (10) zu erhalten.

2. Verfahren nach dem vorhergehenden Anspruch, **dadurch gekennzeichnet, dass** eine dreidimensionale räumliche Verteilung von lokalen Dosen in Echtzeit gemessen wird, die durch eine Mehrzahl von Bereichen (25) einer Zielscheibe (20) empfangen werden, indem die Zielscheibe (20) oder der Detektor (45) in Bezug auf den Detektor (45) bzw. die Zielscheibe (20) verlagert wird, wobei das bidirektionale System zur Detektion von geladenen Teilchen eingesetzt wird.

3. Verfahren nach einem der vorhergehenden Ansprüche, **dadurch gekennzeichnet, dass** in Echtzeit eine longitudinale Verteilung von lokalen Dosen gemessen wird, die durch eine Mehrzahl von Bereichen (45) einer Zielscheibe (20) empfangen werden, indem die Zielscheibe (20) oder der Detektor (45) in Bezug auf den Detektor (45) bzw. auf die Zielscheibe longitudinal versetzt wird.

4. Verfahren nach einem der vorhergehenden Ansprüche, **dadurch gekennzeichnet, dass** in den Strahl von einfallenden Hadronen (10) ein System zur Erfassung von geladenen Teilchen angeordnet wird, welches dazu geeignet ist, eine zeitliche Kennzeichnung der einfallenden Hadronen zu liefern, und entsprechende Parameter in die Messmittel für die Flugzeit der Teilchen eingeführt werden.

5. Verfahren nach einem der vorhergehenden Ansprüche, **dadurch gekennzeichnet, dass** der Abstand L größer oder gleich 40 cm ist, beispielsweise größer oder gleich 60 cm und beispielsweise kleiner oder gleich 100 cm.

6. Verfahren nach einem der Ansprüche der vorhergehenden Ansprüche, **dadurch gekennzeichnet, dass** die Schwellen-Energie Eₛ größer oder gleich 500 keV ist, bevorzugt kleiner oder gleich 5 MeV, ja sogar 2 MeV, beispielsweise in der Größenordnung von 1 MeV.

7. Verfahren nach einem der vorhergehenden Ansprüche, **dadurch gekennzeichnet, dass** der Zeitbereich dt zwischen 1 und 15 ns (Nanosekunden) liegt, beispielsweise in der Größenordnung von 7 ns.

8. Verfahren nach einem der vorhergehenden Ansprüche, **dadurch gekennzeichnet, dass** eine physikalische Visiereinrichtung/Kollimatoreinrichtung (30) mit einem Schlitz (32) zwischen der Zielscheibe (20) und dem Detektor angeordnet wird, welcher den Messmitteln für Energie und Flugzeit (45) zugeordnet ist, oder/und dass der den Messmitteln für Energie und Flugzeit zugeordnete Detektor (45) elektronischen Visiermitteln/Kollimatormitteln zugeordnet ist.

9. Verfahren zur Echtzeit-Messung von einer Mehrzahl von lokalen Dosen, die in einer Mehrzahl von Bereichen (25) einer Zielscheibe (20) empfangen werden, wobei das Verfahren nach einem der Ansprüche 1 bis 8 durchgeführt wird, dank einer Mehrzahl von Detektoren, die Messmitteln für Energie und Flugzeit (45) zugeordnet sind, welche räumlich entlang der oder um die Zielscheibe (20) herum verteilt sind.

10. Vorrichtung zur Echtzeit-Messung von einer lokalen Dosis, die von einem Bereich (25) einer Zielscheibe (20) bei der Bombardierung der Zielscheibe (20) mit einem Strahl von einfallenden Hadronen (10) empfangen wird, umfassend:
- einen Detektor (45) welcher Messmitteln für Energie und Flugzeit von Teilchen zugeordnet ist,
- Visiermittel/Kollimatormittel (30) für den Bereich (25) der Zielscheibe (20),
- Mittel, welche es ermöglichen, Ereignisse auszuwählen, welche Teilchen mit einer Energie E entsprechen, die größer als eine Schwellen-Energie Eₛ ist, aufgezeichnet durch den Detektor (45),
- Mittel, welche es ermöglichen, unter diesen letzteren Ereignissen solche Ereignisse auszuwählen, die in einem gegebenen Zeit-Bereich dt liegen, und wobei der Detektor (45) in einem Abstand L von dem zu messenden Bereich (25) der Zielscheibe (20) angeordnet ist, der so ausgewählt ist, dass ermöglicht wird, schnelle Gammastrahlen und Neutronen dank deren Unterschied in der Ausbreitungsgeschwindigkeit zu unterscheiden,
- ein bidirektionales System zur Detektion von geladenen Teilchen, angeordnet in dem Strahl von einfallenden Hadronen (10) vor der Zielscheibe (20), um die transversale Position der einfallenden Hadronen (10) zu erhalten.

11. Vorrichtung nach dem vorhergehenden Anspruch, **dadurch gekennzeichnet, dass** der Abstand L größer oder gleich 40 cm ist, beispielsweise größer oder gleich 60 cm, und beispielsweise kleiner oder gleich 100 cm.

12. Vorrichtung nach einem der Ansprüche 10 oder 11, **dadurch gekennzeichnet, dass** die Visiermittel/Kollimatormittel eine physikalische Visiereinrichtung/Kollimatoreinrichtung (30) mit einem Schlitz (32) umfassen, die zwischen der Zielscheibe (20) und dem Detektor angeordnet ist, welcher den Messmitteln für Energie und Flugzeit (45) zugeordnet ist, wobei die Achse des Schlitzes (32) senkrecht zu dem Strahl von einfallenden Hadronen (10) ist, oder/und die Visiermittel/Kollimatormittel elektronische Visiermittel/Kollimatormittel umfassen.

13. Vorrichtung nach einem der Ansprüche 10 bis 12, **dadurch gekennzeichnet, dass** der Detektor (45), welcher den Messmitteln für Energie und Flugzeit zugeordnet ist, und die Visiermittel/Kollimatormittel oder/und die Zielscheibe (20) auf Verlagerungsmitteln angeordnet sind, welche eine longitudinale Verlagerung oder/und eine Winkelverlagerung ermöglichen.

14. Vorrichtung zur Echtzeit-Messung von einer Mehrzahl von lokalen Dosen, die von einer Mehrzahl von Bereichen (25) einer Zielscheibe (20) bei der Bombardierung der Zielscheibe (20) mit einem Strahl von einfallenden Hadronen (10) empfangen werden, umfassend eine Mehrzahl von Vorrichtungen nach einem der Ansprüche 10 bis 13, die räumlich entlang der oder/und um die Zielscheibe (20) herum verteilt sind.
